# NEW EUROPEAN PATENT SPECIFICATION

(11) **EP 0 510 949 B2**
(45) Date of publication and mention of the opposition decision: **02.04.2003**
(45) Mention of the grant of the patent: 22.01.1997
(21) Application number: 92303618.0
(22) Date of filing: 22.04.1992
(51) Int. Cl.: A61K 47/48

(54) **Cytomodulating conjugates of members of specific binding pairs**
Zytomodulierte Konjugate enthaltend spezifische Bindungspaargliedern
Conjugués cytomodulateurs constants de pair liants spécifiques

(30) Priority: 23.04.1991 US 690530
(43) Date of publication of application: 28.10.1992
(73) Proprietor: SANGSTAT MEDICAL CORPORATION, Menlo Park, CA 94025 (US)
(72) Inventor: Pouletty, Philippe, Atherton, California 94025 (US)
(74) Representative: Stoner, Gerard Patrick

(56) References cited:
- EP-A- 0 180 171
- EP-A- 0 334 300
- EP-A- 0 352 761
- WO-A-88/03566
- WO-A-90/11779
- WO-A-92/03569
- PROC. NATL. SCI. USA, vol. 88, October 1991, pages 8705-8709, Immunology; H. LIU et al.: "Conventional antigen and superantigen may be coupled to distinct and cooperative T-cell activation pathways"
- STN FILE SERVER, Karlsruhe DE, & FILE CA & CA, vol. 113, no. 7, abstract no. 150283k, Columbus, Ohio, US; M. KOTB et al.: "Accessory cell-independent stimulation of human T cells by streptococcal M protein superantigen"
- Staerz et al, 1986, Proc. Natl. Acad. Sci. USA, 831453-57
- Staerz et al, 1985, Nature, 314; 628-31
- Gravelle et al, 1989, J. Immunol. 142; 4079-4084

## Description

### Description for the following Contracting States : AT, BE, CH, LI, DE, DK, FR, GB, IT, LU, MC, NL, PT, SE

The field of this invention is cytomodulating conjugates of members of specific binding pairs and to therapeutics employing such cytomodulating compounds.

The immune system is our major defense against a wide variety of diseases. However, in many situations, the immune system appears to be unable to protect the host from disease or is aberrant and attacks the host, being unable to distinguish between self and non-self. In both situations, there is an interest in being able to modulate the immune system, either activating the immune system toward a particular target or inactivating the immune system to prevent attack of a target.

For the most part, success in preventing immune system attack has relied upon the total inhbition or suppression of the immune system. In this situation, the host becomes susceptible to a wide variety of opportunistic infections. Therefore, while achieving one goal, one must protect the host against pathogens, which can result in extended periods of hospitalization, maintenance by antibiotics with the resulting side effects, and the like. By contrast, it is believed that the immune system is normally capable of protecting the host from tum-origenesis. However, the substantial incidence of cancer is evidence of the inability of the immune system to maintain perfect surveillance. In this situation, there is an interest in being able to activate the immune system so as to increase its capability to attack cancer cells.

There is, therefore, an interest in providing for therapies, which can be specific for activating particular cells to be directed toward a specific target or inactivating specific cells which are directed to a specific target In this way, one may selectively activate or inactivate cellular members of the immune system to achieve a therapeutic goal.

Lorberbaum et al., J. Biol. Chem. (1990) 265:16311-7 describe a polyethyleneglycol modified IL-2. Batra et al., ibid (1990) 265:15198-202 describe a fusion protein comprising a single chain antibody. Garrido et al., Cancer Res. (1990) 50:4227-32 describe bis-pecific antibodies to target human T-lymphocytes. Pullen et al., Cell (1990) 61:1365-74, describe the region of the T-cell receptor beta chain that interacts with the self-superantigen Mls-1A. Garrido et al., J. Immunol (1990) 144:2891-8 describe targeted cytotoxic cells. Junghans et al., Cancer Res. (1990) 50:1495-502 describe a humanized antibody to the IL-2 receptor. Schroeder et al., Transplantation (1990) 49:48-51 describe antimurine antibody formation following OKT3 therapy. Kaplan and Mazed, Int. J. Artif. Organs (1989) 12:79-804 describe in vitro removal of anti-A and anti-B antibodies with synthetic oligosaccharides. A review of blood groups antigens may be found in FEMS Microbiol. Immunol. (1989) 1:321-30.

Conjugates of selective binding moieties are used to modulate immune response. The conjugates, referred to as "complexines" comprise a moiety which binds to a target cell receptor or receptor complex and a moiety which binds to an effector agent, endogenous to the host, which provides for cytomodulation, normally cytotoxicity. The complexine is administered to the host in amounts sufficient to provide for the desired prophylactic or therapeutic effect.

The present invention provides a conjugate for inactivating a target cell in a mammalian host comprising said target cell and an endogenous cytotoxic effector system comprising at least one effector agent.

The conjugate is introduced into said host in sufficient amount to substantially reduce the target cell population, wherein said conjugate is characterised by consisting essentially of a moiety specific for a surface membrane receptor of said target cell, said moiety being other than an antibody or fragment thereof, joined to a selective moiety capable of binding to said effector system to form a cell inactivating complex,
wherein said effector system comprises (I) antibodies specific for said selective moiety and an antibody dependent cytotoxic system comprising at least one effector agent or (2) a T-cell, with the proviso that when said selective moiety binds to a T-cell, it binds to the T-cell receptor complex,
whereby when said conjugate is bound to said target cell and said effector agent, said target cell is inactivated,
wherein the moiety specific for a surface membrane receptor of said target cell is a cytokine, a ligand for a cluster designation surface membrane protein of said target cell, a growth factor, a ligand that binds an infectious agent, a ligand that binds a bacterial receptor, a ligand that binds a T-cell receptor and a ligand that binds HLA molecules or fragments thereof and the selective moiety is an antigen to which the host has been previously sensitised or to which the host has natural antibodies. The selective moiety may be a blood group antigen or a superantigen. The selective moiety may bind to a cytotoxic T-cell. The moiety for said surface membrane protein may be a ligand for a cytokine surface membrane protein receptor of said target cell. The ligand may be IL-2.

The present invention also provides a conjugate for inactivating a target cell in a mammalian host comprising said target cell:

The conjugate is introduced into said host comprising an endogenous cellular effector system, comprising at least one effector agent, capable of inactivating said target cell, wherein said conjugate is characterised by consisting essentially of a ligand capable of binding to a surface membrane protein receptor, said ligand being other than an antibody or fragment thereof, joined to a selective moiety capable of binding to said effector agent to form a cell inactivating complex, wherein said selective moiety is an antigen to which the host has been previously sensitised or to which the host has natural antibodies and wherein said effector agent comprises an immunoglobulin specific for said selective moiety, whereby when said conjugate is bound to said target cell and said effector agent, said target cell is inactivated. The ligand may be a cytokine such as IL-2.

The present invention also provides a composition consisting essentially of a ligand capable of binding to a surface membrane protein which ligand is other than an antibody or fragment thereof, covalently joined to a blood group antigen or superantigen or at least a portion of a vaccine immunogen. The ligand in the composition may be IL-2 joined to a blood group antigen.

In accordance with the subject invention, methods and compositions are provided for therapeutic treatment of a host, where the action of the immune system is modulated, so as to provide for prophylactic or therapeutic effect. The agent employs a conjugate having two moieties, each moiety having physiological activity. One moiety provides for binding to a target cell. The other moiety provides for interaction with a member of the immune system, whereby endogenous agents provide for the prophylactic or therapeutic effect The conjugates may be as a result of chemical binding, either covalent or non-covalent, or a fusion protein by means of genetic engineering. Thus, the complexine components may be held together by means of a synthetic bridge, a peptide bridge, a membrane, e.g. liposome, polymer or particle, etc.

The conjugates are called "complexines," since they result in the formation of complexes with members of the immune system, which provide for modulation of the activity of a target cell. By administering the complexines to a host, the complexines will bind to a surface membrane protein or protein complex of the target cell, while also binding to endogenous effector agent present in the host. The endogenous effector agent results in an endogenous pathway which provides for inactivation or removal of the target cell from the host For the most part, cytotoxicity is obtained, whereby the target cell is killed.

The moiety which binds to the target Cell receptor may be any of a wide variety of molecules, including ligands for receptors, such as the interleukins, 1-10, particularly -2, -4 and -6; molecules which bind to cluster designation surface membrane proteins, such as CD3, -4, -5, -8, -10, -15, -19, -69, etc.; growth factors, such as GM-CSF, G-CSF, M-CSF, EGF, TGF, TNF, interferons, etc.; molecules which bind to any of the members of the T-cell receptor, either the sub-units of Ti or T3; slg, molecules which bind to infectious agents etc.; molecule which bind to LPS, or other pathogenic cellular marker; molecules which bind to bacterial receptors, etc.; in the case of transplantation of organs, HLA molecules or fragments derived therefrom, derived from donor HLA antigens, particularly the variable region, while for bone marrow transplants the HLA molecules will be from the recipient antigens, etc.

The other moiety of the conjugate is a selective member, where the member directly or indirectly binds selectively to an effector system, comprising one or more effector agents endogenous to the host. By endogenous is intended an agent which is naturally present or may be safely administered to the host, e.g. antibodies, so as to be able to react with the selective members. The member may include an antigen to which the host has been previously sensitized or to which the host has natural antibodies, so as to have memory cells and/or specific soluble antibodies in the blood stream, such as oligosaccharide A or B antigens, vaccine antigens (immunogens) which encounter antibodies due to a prior immune response, e.g. diphtheria or tetanus antitoxin, influenza virus hemagglutinin, HBs antigen, polio virus, rubella virus or measles virus antibodies, such as antibodies to DNA, RNA or ribonucleoprotein; for a T-cell response, tuberculin, HIV, particularly gp 120; a superantigen, such as toxins derived from Staphylococcus or other bacteria, e.g., SEC1, SEA, SBB, EXPT, TSST1, Mls, or minor histocompatibility antigens from mammalian cells. The superantigeris bind to a substantial proportion of the Vβ chains of the T-cell receptor, Ti. In all cases, one may use vicarious groups which provide the same function, e.g. binding. Of particular interest are anti-idiotype antibodies or the variable regions thereof, which will mimic the selective moiety or bind to antibodies present in the host The antibodies may interact with the members of the complement cascade or other cytotoxic agent, e.g. ADCC, to kill the target cell or the selection member may bind to a T-cell which provides a cytotoxic function.

The members of the conjugate may be polypeptides, saccharides, lipids, nucleic acids, or naturally occurring or synthetic organic molecules other than the molecules already described. The members of the conjugate may be joined directly or through a bridge of not more than about 50 members in the chain, usually not more than about 20 members in the chain, where the members of the chain may be carbon, nitrogen, oxygen, sulfur, phosphorous, and the like. Thus, various techniques may be used to join the two members of the conjugate, depending upon the nature of the members of the conjugate, the binding sites of the members of the conjugate, convenience, and the like. Functional groups which may be involved include esters, amides, ethers, phosphates, amino, hydroxy, thio, aldehyde, keto, and the like. The bridge may involve aliphatic, alicyclic, aromatic, or heterocyclic groups. A substantial literature exists for combining organic groups to provide for stable conjugates. Conjugates involving only proteins or glycoproteins can be chimeric or fusion recombinant molecules resulting from expression of ligated open reading frames of natural sequences, synthetic sequences, or combinations thereof.

Illustrative of complexines are IL-2 or CD69 binding protein individually linked to polysaccharide A and polysaccharide B antigen as a mixture, where the complexine comprises individual A and B molecules, although in some situations, it may be desirable to have more than one A or B molecule per conjugate to provide for higher avidity or activity or vary the in vitro solubility of the complex or for extended immune complexes and/or more than one ligand moiety per conjugate, for similar reasons. Since about 95% of individuals have natural anti-A and/or anti-B antibodies, the complexine will be effective in about 95% of individuals. Thus, one could have a combination of IL-2, IL-4 and/or CD69 binding protein or combination of selective moieties e.g. A + B + vaccine Ag.

The multivalent complexine when administered intravenously would result in immune complexes of a size, which allows for solubility in the circulation and will bind to T-cells expressing the target surface membrane protein. If, one wishes to destroy activated T-cells which have a high level of IL-2 receptor or express CD69, the subject complexines will serve to bind via antibodies to the selective moiety to complement or other cytotoxic agent, such as ADCC cells, to substantially reduce the activated T-cell population. The binding of the immune complexes to the target cells will result in complement activation and/or opsonization resulting in target cell lysis. Other effector agens, include lympocytes, or neutrophils, such as T-cells or K-cells, NK cells, monocytes, macrophages, basophils, eosinophils, mastocytes, erythrocytes, etc. By employing superantigen selective for cytotoxic T--cells, one may recruit such cells for their cytotoxic effect.

The subject compositions may be used for the treatment of a wide variety of pathologies, by varying the moiety for the target cell. Thus, treatments may include immunosuppression for organ transplantation, treatment for neoplasia, such as carcinomas, leukemias, lymphomas, sarcomas, melanomas, etc., autoimmune diseases, such as rheumatoid arthritis, multiple sclerosis, lupus, etc.; cellular pathogens; and the like.

One example is immune suppression associated with organ transplantation. In this situation, one would wish to inactivate or destroy T-cells which are active against the organ transplant. Thus, those T-cells which are activated and have high levels of IL-2 receptor or recognize the HLA antigen, may be selectively targeted for destruction by use of a complexine comprising one or more IL-2 ligands or binding portion thereof or other ligands, e.g. other interleukin, or one or more HLA antigens or the variable regions thereof of the organ donor. In the case of a bacterial infection, one may use a lectin or antibody specific for an epitopic site of the pathogen bonded to the A and/or B antigen to enhance the immune response to the pathogen.

The subject conjugates will for the most part be administered parenterally, particularly intravascularly, topically, as an aerosol, orally or the like, depending upon the particular organ, system or chamber to be treated. The amount of the conjugate which is administered will vary widely, depending upon the nature of the conjugate, the nature of the disease being treated, whether one or more administrations are to be made, the endogenous level of the effector or level stimulated by the complexine, the desired cytotoxic level, and the like. Thus, for each conjugate, one will determine empirically the level to be administered for a particular indication. The conjugates may be administered in any convenient carrier, such as distilled water, phosphate buffered saline, saline, aqueous ethanol, blood derivative, or other conventional carrier. Other additives may be included, such as stabilizers, biocides, buffers, salt, and the like, these additives being conventional and used in conventional amounts.

The following examples are by way of above illustration and not by way of limitation.

### EXPERIMENTAL

### Example 1. Complexine with red blood group A antigen.

Blood-group A synthetic trisaccharides (8-azidocarbonyloctyl-derivatives of alphaGalNac1, 3-[alphaFuc 1,2]betaGal, derivatized to include a C-terminal amino group is conjugated to Interleukin 2 as follows:

### I. Activation of Interleukin 2

1. 0.2 mg of (13 nmoles) of IL-2 is dissolved in 0.3 ml of 0.1 M sodium phosphate pH 7.5.
2. 2.3 mg N-Succinimidul S-Acetyl thiolacetate is dissolved in 1 ML of DMSO.
3. 10 µl of N Succinimidyl S-Acetyl thiolacetate is added to the IL-2 solution and the mixture is incubated at 25°C for 30 minutes.
4. The reaction mixture is passed through a G-25 column equilibrated with 0.1 M phosphate buffer pH 6.0. Free thiol groups are introduced by adding 100 µl of 0.5 M hydroxylamine/0.05 M sodium phosphate pH 7.5 containing 0.025M EDTA to the IL2 Solution. The solution is stirred for 120 minutes at room temperature. The solution is passed through a G-25 column and free SH groups on IL-2 are obtained. Free SH groups are quantified using the Ellman's test, by measuring the absorbance at 412 nm.

### II. Activation of Blood Group A Trisaccharide (A antigen) With Maleimido Group

1. A antigen (0.50 to 2 x molar excess of IL-2 is dissolved in 1.0 ml of 0.1 M sodium phosphate pH 7.5.
2. Succinimidyl N-Maleimido-6-aminocaproyl (2-nitro-4-sulfonic acid) phenyl ester. Na (MALSAC-HNSA) (100 molar excess) is dissolved in 20 µl of dimethyl sulfoxide (DMSO).
3. The reagent solution (2) is added to the A antigen solution and the mixture is incubated at 25°C for 1 hour.
4. The reaction is monitored by diluting 10 µl of the reacting mixture into 1.0 mL of 0.01 M sodium phosphate, pH 7 at timed intervals. Each aliquot is analyzed by reading the absorbance at 406 nm (A406) before and after addition of 50 µl of 5 N NAOH. The percentage of active ester at any time is calculated using the formula: [(A406(NaOH) - A406)/A406(NaOH)] x 100. From the difference between the amount of ester at tₒ and at a time thereafter, the amount of ester used at that time is calculated. That corresponds to the amount of total amino group modified.
5. The reaction mixture is centrifuged briefly to remove excess of precipitated reagent and the supernatant is applied to a Sephadex G-25 column equilibrated in 0.1 M phosphate pH 6.0.

### III. Conjugation

6. To maleimido-A antigen (in 0.1 M phosphate pH 6-0), is added IL-2-SH compound and allowed to stir at 4° C overnight. The final molar ratio of Mal-A antigen and IL-2-SH is adjusted from 0.2 to 5. The reaction mixture is then passed through Sephacryl-200. The fractions having peaks at desired mol. weight ranges are collected.

Antigenic reactivity of the conjugate is tested by Western blot using anti-blood group antigen human serum and anti-IL2 monoclonal antibody.

### C: Functional Assay of A-Complexine

In this experiment, it is determined whether the A-Complexine can be used to induce in vitro the specific killing of lymphocytes expressing a high affinity IL-2 receptor, when mixed with human serum containing anti-blood group A antibodies and complement. ⁵¹Cr labelled CTL-L2 lymphocytes are incubated with A-Complexine (from 40 µg/ML to 0.1 ng/mL). After 45 min. incubation at 37° C, human serum containing anti-blood group A antibodies (B group) is added at various dilutions and incubated for 30 minutes at 37° C; Rabbit complement is then added and incubated 1 hour at 37° C. The amount of ⁵¹Cr released is then estimated and the percentage of specific cell lysis calculated. Significant lysis of CTL-L2 cells is observed after incubation with A-Complexine. The phenomenon is dose dependent (increased cytotoxicity), with higher amounts both of A-Complexine and anti-blood group A positive serum) and specific, as IL2-receptor negative cell lines (such as DA-la mouse cells) are not killed under the same assay conditions. Furthermore, no significant cytotoxicity is observed when using human serum from a blood group AB or A individual.

### HBs Complexine

A cyclical peptide derived from the amino-acid sequence (a.a. 139-147) of Hepatitis B virus surface antigen (HBsAg) and showing antigenic reactivity with polyclonal and monoclonal antibodies defining the a epitope of HBsAg (HBs peptide) is used for conjugation with interleukin 2. The peptide sequence is: NH2-Cys-Thr-Lys-Pro-Thr-Asp-Gly-Asn-Cys-Tyr-COOH. It is synthesized by solid phase method (Merrifield) using FMOC chemistry. It is purified by HPLC. A disulfide bond is introduced between the two terminal cysteine residues by oxidation with potassium ferricyanide.

HBs peptide is conjugated to Interleukin 2 as follows:

### I. Activation of Interleukin 2

1. 0.2 mg of (13 nmoles) of IL-2 is dissolved in 0.3 ml of 0.1 M sodium phosphate pH 7.5.
2. 2.3 mg N-Succinimidyl S-Acetyl thiolacetate is dissolved in 1 mL of DMSO.
3. 10 µl of N-Succinimidyl S-Acetyl thiolacetate is added to the IL-2 solution and the mixture is incubated at 25° C for 30 minutes.
4. The reaction mixture is passed through a G-25 column equilibrated with 0.1 M phosphate buffer pH 6.0. Free thiol groups are introduced by adding 100 µl of 0.5 M hydroxylamine/0.05 M sodium phosphate pH 7.5 containing 0.025 M EDTA to the IL-2 solution. The solution is stirred for 120 minutes at room temperature. The solution is passed through a G-25 column and free SH groups on IL-2 are obtained. Free SH groups are quantified using the Ellman's test, by measuring the absorbance at 412 nm.

### II. Activation of HBs Peptide With Maleimido Group

1. HBs peptide (0.50 to 2 x molar excess of IL-2) is dissolved in DMSO at 10 mg/mL and diluted in 1.0 ml of 0.1 M sodium phosphate pH 7.5.
2. Succinimidyl N-Maleimido-6-aminocaproyl (2-nitro-4-sulfonic acid) phenyl ester. Na (MAL-SAC-HNSA) (100 molar excess) is dissolved in 20 µl of N-N dimethyl sulfoxide (DMSO).
3. The reagent solution (2) is added to the HBs peptide solution and the mixture incubated at 25° C for 1 hour.
4. The reaction is monitored by diluting 10 µl of the reaction mixture into 1.0 ML of 0.01 M sodium phosphate pH 7 at timed intervals. Each aliquot is analyzed by reading the absorbance at 406 nm (A406) before and after addition of 50 µl of 5N NAOH. The percentage of active ester at any time is calculated using the formula: [(A406(NaOH) - A406)/A406(Na/OH)] x 100. From the difference between the amount of ester present at tₒ and at a time thereafter, the amount of ester used at that time is calculated. That corresponds to the amount of total amino group modified.
5. The reaction mixture is centrifuged briefly to remove the excess of precipitated reagent and the supernatant is applied to a Sephadex (G-25 column equilibrated in 0.1 M phosphate pH 6.0).

### III. Conjugation

6. To maleimido-HBs peptide (in 0.1 M Phosphate pH 6.0) is added II-2-SH compound and allowed to stir at 4° C overnight. The final molar ratio of Mal-HBs peptide and IL₂-SH are adjusted from 0.2 to 5. Finally the reaction mixture is passed through Sephacryl-200. The peaks at the desired mol. weight ranges are collected.

Antigenic reactivity of the conjugate is tested by Western blot using anti-HBs monoclonal antibody (A specific) and anti-IL-2 monocolonal antibody.

### C: Functional Assay of HBs-Complexine:

In this experiment, it is determined whether the HBs-Complexine can be used to induce in vitro the specific killing of lymphocytes expressing a high affinity IL-2 receptor, when mixed with human serum containing anti-HBs antibodies and complement. ⁵¹Cr labelled CTL-L2 lymphocytes are incubated with HBs-Complexine (from 20 µg/mL to 0.1 ng/mL). After 45 min. incubation at 37 °C, human serum containing anti-HBs antibodies (collected from a patient vaccinated using Hevac B, Pasteur Vaccins and tested for anti-HBs antibodies by ELISA (Abbott Laboratories)) is added at various dilutions, incubated for 30 minutes at 37°C; Rabbit complement is then added and incubated 1 hour at 37 ° C. The amount of ⁵¹Cr released is then estimated and the percentage of specific cell lysis calculated. Significant lysis of CTL-L2 cells is observed after incubation with HBs-Complexine. The phenomenon is dose dependent: increased cytotoxicity is observed both with higher amounts of HBs-Complexine and anti-HBs positive serum. Cytotoxicity is specific, as IL-2 receptor negative cell lines (such as DA-Ia mouse cells) are not killed under the same assay conditions. Furthermore, no significant cytotoxicity is observed when using human serum negative for anti-HBs antibodies.

In accordance with the subject invention, agents are provided which can specifically bind to a target cell, human, bacteria, virus infected or parasitic, via a specific binding ligand. Thus, agents can be selected which show a low affinity for cells which do not pair with the receptor complementary binding member. While using specific agents which interact with an endogenous effector molecule, one can achieve cytotoxicity toward the target cell following binding of the conjugate to the target cell. By employing agents for the ligand moiety, which do not induce a significant immune response, such as molecules which are substantially endogenous or have low immunogenicity, one can avoid an immune response and thus avoid having agents of the immune response destroy or inactivate the therapeutic conjugate. In contrast, by taking advantage of the preexisting immune response against the selective moiety and because the ligand moiety remains functional, one can use the subject agents on a chronic basis.

All publications and patent applications mentioned in this specification are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the scope of the appended claims.

## Claims (Claims for the following Contracting State(s): AT, BE, CH, LI, DE, DK, FR, GB, IT, LU, MC, NL, PT, SE)

1. A conjugate for inactivating a target cell in a mammalian host which has an endogenous cytotoxic effector system comprising at least one effector agent, said conjugate consisting essentially of a moiety specific for a surface membrane receptor of said target cell, said moiety being other than an antibody or fragment thereof, joined to a selective moiety capable of binding to said effector system to form a cell inactivating complex,
wherein said effector system comprises (1) antibodies specific for said selective moiety and an antibody dependent cytotoxic system comprising at least one effector agent or (2) a T-cell, with the proviso that when said selective moiety binds to a T-cell, it binds to the T-cell receptor complex,
whereby when said conjugate is bound to said target cell and said effector agent, said target cell is inactivated,
wherein the moiety specific for a surface membrane receptor of said target cell is a cytokine, a ligand for a cluster designation surface membrane protein of said target cell, a growth factor, a ligand that binds an infectious agent, a ligand that binds a bacterial receptor, a ligand that binds a T-cell receptor and a ligand that binds HLA molecules or fragments thereof and the selective moiety is an antigen to which the host has been previously sensitised or to which the host has natural antibodies.

2. A conjugate according to claim 1, wherein said selective moiety is a blood group antigen or a superantigen.

3. A conjugate according to claim 1, wherein said selective moiety binds to a cytotoxic T-cell.

4. A conjugate according to claim 1, wherein said moiety for said surface membrane protein is a ligand for a cytokine surface membrane receptor of said target cell.

5. A conjugate according to claim 4, wherein said ligand is IL-2.

6. A conjugate for inactivating a target cell in a mammalian host which has an endogenous cellular effector system comprising at least one effector agent, said conjugate consisting essentially of a ligand capable of binding to a surface membrane protein receptor, said ligand being other than an antibody or fragment thereof, joined to a selective moiety capable of binding to said effector agent to form a cell inactivating complex, wherein said selective moiety is an antigen to which the host has been previously sensitised or to which the host has natural antibodies
and wherein said effector agent comprises an immunoglobulin specific for said selective moiety, whereby when said conjugate is bound to said target cell and said effector agent, said target cell is inactivated.

7. A conjugate according to claim 6, wherein said ligand is a cytokine.

8. A conjugate according to claim 7, wherein said ligand is IL-2.

9. A pharmaceutical comprising a conjugate according to any one of claims 1 to 8.

10. The use of a conjugate according to any one of claims 1 to 8 in the preparation of a medicament for inactivating a target cell in a mammalian host.

11. A composition consisting essentially of a ligand capable of binding to a surface membrane protein which ligand is other than an antibody or fragment thereof, covalently joined to a blood group antigen or superantigen or at least a portion of a vaccine immunogen.

12. A composition according to claim 11, wherein said moiety is IL-2 joined to a blood group antigen.

13. A nucleotide sequence which encodes a conjugate according to any one of claims 1 to 8 where such conjugate is a protein.

## Claims (Claims for the following Contracting State(s): ES)

1. A method comprising the preparation of conjugate for inactivating a target cell in a mammalian host which has an endogenous cytotoxic effector system comprising at least one effector agent, said conjugate consisting essentially of a moiety specific for a surface membrane receptor of said target cell, said moiety being other than an antibody or fragment thereof, joined to a selective moiety capable of binding to said effector system to form a cell inactivating complex,
wherein said effector system comprises (1) antibodies specific for said selective moiety and an antibody dependent cytotoxic system comprising at least one effector agent or (2) a T-cell, with the proviso that when said selective moiety binds to a T-cell, it binds to the T-cell receptor complex,
whereby when said conjugate is bound to said target cell and said effector agent, said target cell is inactivated,
wherein the moiety specific for a surface membrane receptor of said target cell is a cytokine, a ligand for a cluster designation surface membrane protein of said target cell, a growth factor, a ligand that binds an infectious agent, a ligand that binds a bacterial receptor, a ligand that binds a T-cell receptor and a ligand that binds HLA molecules or fragments thereof and the selective moiety is an antigen to which the host has been previously sensitised or to which the host has natural antibodies.

2. A method according to claim 1, wherein said selective moiety is a blood group antigen or a superantigen.

3. A method according to claim 1, wherein said selective moiety binds to a cytotoxic T-cell.

4. A method according to claim 1, wherein said moiety for said surface membrane protein is a ligand for a cytokine surface membrane receptor of said target cell.

5. A method according to claim 4, wherein said ligand is IL-2.

6. A method comprising the preparation of a conjugate for inactivating a target cell in a mammalian host which has an endogenous cellular effector system comprising at least one effector agent, said conjugate consisting essentially of a ligand capable of binding to a surface membrane protein receptor, said ligand being other than an antibody or fragment thereof, joined to a selective moiety capable of binding to said effector agent to form a cell inactivating complex, wherein said selective moiety is an antigen to which the host has been previously sensitised or to which the host has natural antibodies
and wherein said effector agent comprises an immunoglobulin specific for said selective moiety, whereby when said conjugate is bound to said target cell and said effector agent, said target cell is inactivated.

7. A method according to claim 6, wherein said ligand is a cytokine.

8. A method according to claim 7, wherein said ligand is IL-2.

9. A method comprising the preparation of a pharmaceutical comprising a conjugate according to any one of claims 1 to 8.

10. The use of a conjugate according to any one of claims 1 to 8 in the preparation of a medicament for inactivating a target cell in a mammalian host.

11. A method for the preparation of a composition consisting essentially of a ligand capable of binding to a surface membrane protein which ligand is other than an antibody or fragment thereof, covalently joined to a blood group antigen or superantigen or at least a portion of a vaccine immunogen.

12. A method according to claim 11, wherein said moiety is IL-2 joined to a blood group antigen.

13. A method comprising obtaining a nucleotide sequence which encodes a conjugate according to any one of claims 1 to 8 where such conjugate is a protein.

## Patentansprüche (Patentansprüche für folgende(n) Vertragsstaat(en): AT, BE, CH, LI, DE, DK, FR, GB, IT, LU, MC, NL, PT, SE)

1. Konjugat zum Inaktivieren einer Zielzelle in einem Säugetierwirt, der ein endogenes zytotoxisches Effektorsystem aufweist, das zumindest ein Effektormittel umfaßt, wobei das Konjugat im wesentlichen aus einer Gruppe besteht, die für einen Oberflächenmembran-Rezeptor der Zielzelle spezifisch ist, wobei die Gruppe kein Antikörper oder Fragment davon ist, und die mit einer selektiven Gruppe verbunden ist, die sich an das Effektorsystem binden kann, um einen Zellinaktivierungskomplex zu bilden,
worin das Effektorsystem umfaßt: (1) Antikörper, die für die selektive Gruppe spezifisch sind, und ein antikörperabhängiges zytotoxisches System, das zumindest ein Effektormittel umfaßt, oder (2) eine T-Zelle, mit der Maßgabe, daß, wenn sich die selektive Gruppe an eine T-Zelle bindet, sie sich an den T-Zellen-Rezeptorkomplex bindet,
wodurch, wenn das Konjugat an die Zielzelle und das Effektormittel gebunden ist, die Zielzelle inaktiviert ist,
worin die für einen Oberflächenmembran-Rezeptor der Zielzelle spezifische Gruppe ein Zytokin, ein Ligand für ein Oberflächenmembran-Protein mit Clusterbezeichnung der Zielzelle, ein Wachstumsfaktor, ein Ligand, der sich an einen Infektionserreger bindet, ein Ligand, der sich an einen bakteriellen Rezeptor bindet, ein Ligand, der sich an einen T-Zellenrezeptor bindet, und ein Ligand ist, der HLA-Moleküle oder Fragmente davon bindet, und die selektive Gruppe ein Antigen ist, für das der Wirt zuvor sensibilisiert worden ist oder für den der Wirt natürliche Antikörper aufweist.

2. Konjugat nach Anspruch 1, worin die selektive Gruppe ein Blutgruppenantigen oder ein Superantigen ist.

3. Konjugat nach Anspruch 1, worin sich die selektive Gruppe an eine zytotoxische T-Zelle bindet.

4. Konjugat nach Anspruch 1, worin die Gruppe für das Oberflächenmembranprotein ein Ligand für einen Zytokin-Oberflächenmembran-Rezeptor der Zielzelle ist.

5. Konjugat nach Anspruch 4, worin der Ligand IL-2 ist.

6. Konjugat zum Inaktivieren einer Zielzelle in einem Säugetierwirt, der ein endogenes zellulares Effektorsystem aufweist, das zumindest ein Effektormittel umfaßt, wobei das Konjugat im wesentlichen aus einem Liganden besteht, der sich an einen Oberflächenmembran-Proteinrezeptor binden kann, wobei der Ligand kein Antikörper oder Fragment davon ist, und der mit einer selektiven Gruppe verbunden ist, die sich an das Effektormittel binden kann, um einen Zellinaktivierungskomplex zu bilden, worin die selektive Gruppe ein Antigen ist, für das der Wirt zuvor sensibilisiert worden ist oder gegen den der Wirt natürliche Antikörper aufweist,
und worin das Effektormittel ein Immunglobulin umfaßt, das für die selektive Gruppe spezifisch ist, wodurch, wenn das Konjugat an die Zielzelle und das Effektormittel gebunden ist, die Zielzelle inaktiviert ist.

7. Konjugat nach Anspruch 6, worin der Ligand ein Zytokin ist.

8. Konjugat nach Anspruch 7, worin der Ligand IL-2 ist.

9. Pharmazeutikum, das ein Konjugat nach einem der Ansprüche 1 bis 8 umfaßt.

10. Verwendung eines Konjugats nach einem der Ansprüche 1 bis 8 zur Herstellung eines Medikaments zur Inaktivierung einer Zielzelle in einem Säugetierwirt.

11. Zusammensetzung, die im wesentlichen aus einem Liganden besteht, der sich an ein Oberflächenmembran-Protein binden kann, wobei der Ligand kein Antikörper oder Fragment davon ist, und der kovalent mit einem Blutgruppenantigen oder einem Superantigen oder zumindest mit einem Teil eines Impfstoffimmunogens verbunden ist.

12. Zusammensetzung nach Anspruch 11, worin die Gruppe IL-2 ist, das mit einem Blutgruppenantigen verbunden ist.

13. Nukleotidsequenz, die für ein Konjugat nach einem der Ansprüche 1 bis 8 kodiert, worin ein solches Konjugat ein Protein ist.

## Patentansprüche (Patentansprüche für folgende(n) Vertragsstaat(en): ES)

1. Verfahren, umfassend die Herstellung eines Konjugats zum Inaktivieren einer Zielzelle in einem Säugetierwirt, der ein endogenes zytotoxisches Effektorsystem aufweist, das zumindest ein Effektormittel umfaßt, wobei das Konjugat im wesentlichen aus einer Gruppe besteht, die für einen Oberflächenmembran-Rezeptor der Zielzelle spezifisch ist, wobei die Gruppe kein Antikörper oder Fragment davon ist, und die mit einer selektiven Gruppe verbunden ist, die sich an das Effektorsystem binden kann, um einen Zellinaktivierungskomplex zu bilden,
worin das Effektorsystem umfaßt: (1) Antikörper, die für die selektive Gruppe spezifisch sind, und ein antikörperabhängiges zytotoxisches System, das zumindest ein Effektormittel umfaßt, oder (2) eine T-Zelle, mit der Maßgabe, daß, wenn sich die selektive Gruppe an eine T-Zelle bindet, sie sich an den T-Zellen-Rezeptorkomplex bindet,
wodurch, wenn das Konjugat an die Zielzelle und das Effektormittel gebunden ist, die Zielzelle inaktiviert ist,
worin die für einen Oberflächenmembran-Rezeptor der Zielzelle spezifische Gruppe ein Zytokin, ein Ligand für ein Oberflächenmembran-Protein mit Clusterbezeichnung der Zielzelle, ein Wachstumsfaktor, ein Ligand, der sich an einen Infektionserreger bindet, ein Ligand, der sich an einen bakteriellen Rezeptor bindet, ein Ligand, der sich an einen T-Zellenrezeptor bindet, und ein Ligand ist, der HLA-Moleküle oder Fragmente davon bindet, und die selektive Gruppe ein Antigen ist, für das der Wirt zuvor sensibilisiert worden ist oder für den der Wirt natürliche Antikörper aufweist.

2. Verfahren nach Anspruch 1, worin die selektive Gruppe ein Blutgruppenantigen oder ein Superantigen ist.

3. Verfahren nach Anspruch 1, worin sich die selektive Gruppe an eine zytotoxische T-Zelle bindet.

4. Verfahren nach Anspruch 1, worin die Gruppe für das Oberflächenmembranprotein ein Ligand für einen Zytokin-Oberflächenmembran-Proteinrezeptor der Zielzelle ist.

5. Verfahren nach Anspruch 4, worin der Ligand IL-2 ist.

6. Verfahren, umfassend die Herstellung eines Konjugats zum Inaktivieren einer Zielzelle in einem Säugetierwirt, der ein endogenes zellulares Effektorsystem aufweist, das zumindest ein Effektormittel umfaßt, wobei das Konjugat im wesentlichen aus einem Liganden besteht, der sich an einen Oberflächenmembran-Proteinrezeptor binden kann, wobei der Ligand kein Antikörper oder Fragment davon ist, und der mit einer selektiven Gruppe verbunden ist, die sich an das Effektormittel binden kann, um einen Zellinaktivierungskomplex zu bilden, worin die selektive Gruppe ein Antigen ist, für das der Wirt zuvor sensibilisiert worden ist oder gegen den der Wirt natürliche Antikörper aufweist,
und worin das Effektormittel ein Immunglobulin umfaßt, das für die selektive Gruppe spezifisch ist, wodurch, wenn das Konjugat an die Zielzelle und das Effektormittel gebunden ist, die Zielzelle inaktiviert ist.

7. Verfahren nach Anspruch 6, worin der Ligand ein Zytokin ist.

8. Verfahren nach Anspruch 7, worin der Ligand IL-2 ist.

9. Verfahren, umfassend die Herstellung eines Pharmazeutikums, das ein Konjugat nach einem der Ansprüche 1 bis 8 umfaßt.

10. Verwendung eines Konjugats nach in einem der Ansprüche 1 bis 8 zur Herstellung eines Medikaments zur Inaktivierung einer Zielzelle in einem Säugetierwirt.

11. Verfahren zur Herstellung einer Zusammensetzung, die im wesentlichen aus einem Liganden besteht, der sich an ein Oberflächenmembran-Protein binden kann, wobei der Ligand ein Antikörper oder Fragment davon ist, und der kovalent mit einem Blutgruppenantigen oder einem Superantigen oder zumindest mit einem Teil eines Impfstoffimmunogens verbunden ist.

12. Verfahren nach Anspruch 11, worin die Gruppe IL-2 ist, das mit einem Blutgruppenantigen verbunden ist.

13. Verfahren, umfassend das Erhalten einer Nukleotidsequenz, die für ein Konjugat nach einem der Ansprüche 1 bis 8 kodiert, worin ein solches Konjugat ein Protein ist.

## Revendications (Revendications pour l'(les) Etat(s) contractant(s) suivant(s): DE, DK, FR, GB, IT, LU, MC, NL, PT, SE)

1. Conjugué pour inactiver une cellule cible chez un hôte mammalien qui a un système effecteur cytotoxique endogène, comprenant au moins un agent effecteur, ledit conjugué comprenant une entité spécifique d'un récepteur de membrane de surface de ladite cellule cible, ledit fragment étant autre qu'un anticorps ou son fragment, joint à un fragment sélectif capable de se lier audit système effecteur pour former un complexe inactivant la cellule,
où ledit système effecteur comprend (1) des anticorps spécifiques du fragment sélectif et un système cytotoxique dépendant de l'anticorps comprenant au moins un agent effecteur ou (2) une cellule T, à condition que quand ledit fragment sélectif se lie à une cellule T, il se lie au complexe du récepteur de la cellule T,
ainsi, quand ledit conjugé est lié à ladite cellule cible et audit agent effecteur, ladite cellule cible est inactivée,
où le fragment spécifique d'un récepteur de membrane de surface de ladite cellule cible est une cytokine, un ligand pour une protéine de membrane de surface de désignation d'amas de ladite cellule cible, un facteur de croissance, un ligand qui lie un agent infectieux, un ligand qui lie un récepteur bactérien, un ligand qui lie un récepteur de cellule T et un ligand qui lie des molécules HLA ou leurs fragments et le fragment sélectif est un antigel auquel l'hôte a au préalable été sensibilisé, ou auquel l'hôte a des anticorps naturels.

2. Conjugué selon la revendication 1, où ledit fragment sélectif est un antigène du groupe sanguin ou un super-antigène.

3. Conjugué selon la revendication 1, où ledit fragment sélectif se lie à une cellule T cytotoxique.

4. Conjugué selon la revendication 1, où ledit fragment pour ladite protéine de membrane de surface est un ligand pour un récepteur de protéine de membrane de surface de cytoxine de ladite cellule cible.

5. Conjugué selon la revendication 4, où ledit ligand est IL-2.

6. Conjugué pour inactiver une cellule cible chez un hôte mammalien qui a un système effecteur cellulaire endogène comprenant au moins un agent effecteur, ledit conjugué comprenant un ligand capable de se lier à un récepteur de protéine de membrane de surface, ledit ligand étant autre qu'un anticorps ou son fragment joint, à un fragment sélectif capable de se lier audit agent effecteur, pour former un complexe inactivant la cellule, où ledit fragment sélectif est un antigène auquel l'hôte a au préalable été sensibilisé ou auquel l'hôte a des anticorps naturels
et, où ledit agent effecteur comprend une immunoglobuline, spécifique dudit fragment sélectif, ainsi quand ledit conjugué est lié à ladite cellule cible et audit agent effecteur, ladite cellule cible est inactivée.

7. Conjugué selon la revendication 6, où ledit ligand est une cytokine.

8. Conjugué selon la revendication 7, où ledit ligand est IL-2.

9. Produit pharmaceutique comprenant un conjugué selon l'une des revendications 1 à 8.

10. Utilisation d'un conjugué selon l'une des revendications 1 à 8 dans la préparation d'un médicament pour inactiver une cellule cible chez un hôte mammalien.

11. Composition consistant essentiellement en un ligand capable de se lier à une protéine de membrane de surface, lequel ligand est autre qu'un anticorps ou son fragment, joint de manière covalente à un antigène de groupe sanguin ou super-antigène ou au moins une portion d'un immunogène de vaccin.

12. Composition selon la revendication 11, où ledit fragment est IL-2 jointe à un antigène du groupe sanguin.

13. Séquence de nucléotides qui code pour un conjugué selon l'une quelconque des revendications 1 à 8, où ledit conjugué est une protéine.

## Revendications (Revendications pour l'(les) Etat(s) contractant(s) suivant(s): ES)

1. Méthode comprenant la préparation d'un conjugué pour inactiver une cellule cible chez un hôte mammalien qui a un système effecteur citotoxique endogène comprenant au moins un agent effecteur, ledit conjugué comprenant une entité spécifique d'un récepteur de membrane de surface de ladite cellule cible, ledit fragment étant autre qu'un anticorps
ou son fragment, joint à un fragment sélectif capable de se lier audit système effecteur pour former un complexe inactivant la cellule,
où ledit système effecteur comprend (1) des anticorps spécifiques du fragment sélectif et un système cytotoxique dépendant de l'anticorps comprenant au moins un agent effecteur ou (2) une cellule T, à condition que quand ledit fragment sélectif se lie à une cellule T, il se lie au complexe du récepteur de la cellule T,
ainsi, quand ledit conjugé est lié à ladite cellule cible et audit agent effecteur, ladite cellule cible est inactivée,
où le fragment spécifique d'un récepteur de membrane de surface de ladite cellule cible est une cytokine, un ligand pour protéine de membrane de surface de désignation d'amas de ladite cellule cible, un facteur de croissance, un ligand qui lie un agent infectieux, un ligand qui lie un récepteur bactérien, un ligand qui lie un récepteur de cellule T et un ligand qui lie des molécules HLA ou leurs fragments et le fragment sélectif est un antigène auquel l'hôte a au préalable été sensibilisé, ou auquel l'hôte a des anticorps naturels.

2. Méthode selon la revendication 1 où ledit fragment sélectif est un antigène du groupe sanguin ou super-antigène.

3. Méthode selon la revendication 1, où ledit fragment sélectif se lie à une cellule T cytotoxique.

4. Méthode selon la revendication 1, où ledit fragment pour ladite protéine de membrane de surface est un ligand pour un récepteur de protéine de membrane de surface de cytoxine de ladite cellule cible.

5. Méthode selon la revendication 4, où ledit ligand est IL-2.

6. Méthode comprenant la préparation d'un conjugué pour inactiver une cellule cible chez un hôte mammalien qui a un système effecteur cellulaire endogène comprenant au moins un agent effecteur, ledit conjugué consistant essentiellement en un ligand capable de se lier à un récepteur de protéine de membrane de surface, ledit ligand étant autre qu'un anticorps ou son fragment, joint à un fragment sélectif capable de se lier audit agent effecteur, pour former un complexe inactivant une cellule, où ledit fragment sélectif est un antigène auquel l'hôte a été au préalable sensibilisé ou auquel l'hôte a des anticorps naturels
et où ledit agent effecteur comprend une immunoglobuline spécifique dudit fragment sélectif, ainsi quand ledit conjugué est lié à ladite cellule cible et audit agent effecteur, ladite cellule cible est inactivée.

7. Méthode selon la revendication 6, où ledit ligand est une cytokine.

8. Méthode selon la revendication 7, où ledit ligand est IL-2.

9. Méthode comprenant la préparation d'un produit pharmaceutique comprenant un conjugué selon l'une des revendications 1 à 8.

10. Utilisation d'un conjugué selon l'une des revendications 1 à 8 dans la préparation d'un médicament pour inactiver une cellule cible chez un hôte mammalien.

11. Méthode pour la préparation d'une composition consistant essentiellement en un ligand capable de se lier à une protéine de membrane de surface, lequel ligand est autre qu'un anticorps ou son fragment, joint de manière covalente à un antigène du groupe sanguin ou super-antigène ou au moins une portion d'un immunogène de vaccin.

12. Méthode selon la revendication 11, où ledit fragment est IL-2 jointe à un antigène du groupe sanguin.

13. Méthode consistant à obtenir une séquence de nucléotides qui code pour un conjugué selon l'une quelconque des revendications 1 à 8, où ledit conjugué est une protéine.
